# EUROPEAN PATENT APPLICATION

(11) **EP 1 886 692 A1**
(43) Date of publication of application: **13.02.2008**
(21) Application number: 06746919.7
(22) Date of filing: 26.05.2006
(51) Int. Cl.: A61K 45/00, A61K 38/04, A61K 38/22, A61P 3/04, A61P 3/06, A61P 3/10, A61P 9/00, A61P 9/10, A61P 9/12

(54) **AGENT FOR IMPROVEMENT OF INSULIN RESISTANCE**

(30) Priority: 27.05.2005 JP 2005155312
(71) Applicant: Asubio Pharma Co., Ltd., Minato-ku Tokyo 107-8541 (JP)
(72) Inventor: WAKABAYASHI, Naomi BioPharma Center, ASUBIO PHARMA CO., ltd., Gunma, 370-0603 (JP); HARADA, Yuriko, 3740041 (JP); MASUDA, Yutaka BioPharma Center, ASUBIO PHARMA CO., ltd., Gunma, 370-0603 (JP)
(74) Representative: Hiebl, Inge Elisabeth
(86) International application number: PCT/JP2006/310590
(87) International publication number: WO 2006/126688

(57) **Abstract**

A novel dosage regimen of GLP-1 receptor agonists causes little or no side effects or drug interactions and is suitable for improving insulin resistance. Also provided is an insulin resistance improver for use in the dosage regimen. The dosage regimen comprises repeatedly administering, preferably in a non-invasive manner, a GLP-1 receptor agonist at least before eating for a predetermined period of time to create a condition similar to what is observed with postprandial temporary secretion of the endogenous GLP-1 receptor agonist, rather than administering it continuously. This creates a similar or enhanced variation in the plasma levels of GLP-1 receptor agonist as compared to the circadian variation of the endogenous GLP-1 receptor agonist in a healthy individual. A pharmaceutical composition for use in the dosage regimen containing GLP-1 receptor agonist as an active ingredient is also provided.

SEQUENCE LISTING

<110> ASUBIO PHARMA CO., LTD.

<120> Agent for improvement of insulin resistance

<130> 18199EP

<140> 06 746 919.7
<141> 2006-05-26

<150> JP 2005-155312
<151> 2005-05-27

<160> 1

<170> PatentIn version 3.1

<210> 1
<211> 37
<212> PRT
<213> Bos taurus

<220>
<221> PEPTIDE
<222> (1)..(37)
<223> Amino acid sequence for Glucagon-like peptide

<400> 1
His Asp Glu Phe Glu Arg His Ala Glu Gly Thr Phe Thr Ser Asp Val
1               5                   10                  15
Ser Ser Tyr Leu Glu Gly Gln Ala Ala Lys Glu Phe Ile Ala Trp Leu Val
            20                  25                  30
Lys Gly Arg Gly
    35

## Description

### TECHNICAL FIELD

The present invention relates to an insulin resistance improver and its use.

### TECHNICAL BACKGROUND

Insulin resistance is a condition in which tissues such as muscle, liver and fat in the body respond poorly to insulin. Upon onset of insulin resistance, the insulin sensitivity of tissues decreases, resulting in a decrease in the insulin-dependent glucose uptake. As a result, the ability of insulin to decrease blood glucose levels is reduced. This leads to compensatory hyperinsulinemia or, when insulin secretion is insufficient, hyperglycemia (Non-Patent Document 1).

In particular, decreased insulin secretion and insulin resistance are independent factors for the decreased insulin levels in patients with type-2 diabetes and are in fact what define the nature of the disease. One effective treatment for type-2 diabetes is the use of insulin resistance improvers that increase the insulin-dependent glucose uptake by the tissue. Many drugs of this type have been developed and are now in clinical use (Non-Patent Document 2).

However, not a small number of the currently used insulin resistance improvers are known to cause serious side effects that can make the treatment difficult. In fact, several cases have been reported in which side effects such as liver function failure, edema, body weight gain and an increase in adipose tissue weight occurred, resulting in the ban of using or selling these drugs. The concern about the side effects of insulin resistance improvers limits their application (Non-Patent Document 2).

GLP-1(7-36) amide, a glucagon-like peptide-1 (GLP-1) receptor agonist, has attracted much attention as a new approach for treating type-2 diabetes (Patent Documents 1 to 7; Non-Patent Documents 4, 5 and 6). GLP-1(7-36) amide is a polypeptide hormone secreted from L-cells in the lower small intestine. The hormone is secreted temporarily after each meal and acts directly or indirectly on pancreas or other tissues in the body. GLP-1(7-36) amide is known to stimulate insulin secretion in a glucose-dependent manner, suppress glucagon secretion, suppress appetite, and decrease gastrointestinal motility (Non-Patent Document 7). It is believed that GLP-1(7-36) amide serves to make the body ready for the next food intake while regulating the absorption of dietary glucose and blood glucose levels (Non-Patent Document 8).

While patients with type-2 diabetes have a decreased plasma concentration of GLP-1(7-36) amide, their pancreatic beta cells retain the ability to respond to GLP-1(7-36) amide (Non-Patent Document 9). Thus, replacing the depleted endogenous GLP-1(7-36) amide is expected to ameliorate the conditions of type-2 diabetes.

However, GLP-1(7-36) amide, a peptide, is immediately metabolized by a digestive enzyme dipeptidyl peptidase (DPP)-IV into an inactive metabolite (GLP-1(9-36) amide) once administered to the body (Non-Patent Document 10). In addition, the chemical properties of GLP-1(7-36) amide as a peptide limit its administration route to injection or similar invasive methods, making it considerably difficult to use GLP-1(7-36) amide as a treatment for chronic diseases such as type-2 diabetes.

For these reasons, the following approaches have been used to exploit the biological activity of GLP-1(7-36) amide in treating type-2 diabetes (Non-Patent Documents 4, 5 and 6):
(1) administer by injection or other invasive methods a long-acting GLP-1 receptor agonist that has the same activity as GLP-1(7-36) amide and is resistant to DPP-IV degradation.
(2) continuously administer GLP-1(7-36) amide either intravenously or subcutaneously by using a special device such as perfusion pump.
(3) administer an inhibitor of DPP-IV, the digestive enzyme of GLP-1(7-36) amide, to prevent the endogenous GLP-1(7-36) amide secreted after eating from being metabolized into the inactive metabolite GLP-1(9-36) amide, thereby maintaining the plasma concentration of GLP-1(7-36) amide.

Nevertheless, the treatment using a long-acting GLP-1 receptor agonist (1) (Non-Patent Documents 11, 12 and 13) and the treatment by continuous administration of GLP-1(7-36) amide (2) (Non-Patent Documents 8 and 9) each involve the long-term use of GLP-1 receptor agonists at significantly higher concentrations than the endogenous levels of GLP-1(7-36) amide and may thus interrupt the diverse biological activities of GLP-1(7-36) amide. Specifically, how the plasma levels of GLP-1 receptor agonists vary during the course of these treatments is quite different from the manner that the plasma levels of endogenous GLP-1 receptor agonists vary, which occurs due to a temporary rise after each meal. This can induce desensitization of GLP-1 receptors and, thus, attenuated activity of GLP-1(7-36) amide. As a result, the biological activities of GLP-1(7-36) amide are not fully exploited.

The treatment using a DPP-IV inhibitor (3) (Non-Patent Documents 14 and 15) is associated with problems resulting from the selectivity of DPP-IV. In addition to GLP-1(7-36) amide, DPP-IV is known to be involved in the metabolism of many other hormones, including GLP-2, GIP, GHRH and PYY (Non-Patent Document 16). For this reason, not all of the pharmacological effects that appear in diabetic patients or animal models after administration of DPP-IV inhibitors are attributable to the decreased degradation of GLP-1(7-36) amide (Non-Patent Documents 17, 18 and 19). Furthermore, the non-selective, long-term inhibition of DPP-IV by a DPP-IV inhibitor may cause side effects unexpected from animal tests.

Also known as CD26, DPP-IV is deeply involved in the regulation of immune cell activity. It plays a particularly crucial role in the T-cell activity (Non-Patent Document 20). This suggests that the risk that administration of DPP-IV inhibitors can cause reduced immune function. One studies reports that repeated administration of a DPP-IV inhibitor induces a compensatory increase in the DPP-IV activity and, consequently, the activity of the inhibitor decreases (Non-Patent Document 18).
None of the above-described treatments reveals whether GLP-1 receptor agonists have an ability to directly improve the insulin resistance or directly enhance the insulin sensitivity of patients.

In a test in which a GLP-1 receptor agonist was administered to diabetic animal models, the hyperglycemia was ameliorated, which in turn brought about an improvement in the insulin resistance as a secondary effect (Non-Patent Document 21). In another test, the diabetic condition was improved or progressing thereof was stopped, which improved, or prevented worsening of, reactivity of blood vessels (Non-Patent Document 22). The results of these tests are interpreted as mere secondary effects of the traditionally known biological activity of GLP-1 receptor agonists, such as stimulation of insulin secretion and suppression of appetite: Maintaining high plasma concentrations of GLP-1 receptor agonists for a prolonged period of time serves primarily to decrease the blood glucose levels. The fall in the blood glucose levels then leads to a secondary effect in the form of an improvement in insulin resistance or the reactivity of blood vessels. Thus, it is considered that GLP-1 receptor agonists can induce more significant secondary effects when used for a long period of time at a high concentration.

As described above, although GLP-1 receptor agonists, especially GLP-1(7-36) amide, have been proven effective in the treatment of diabetes, no clinically effective approach has thus far been developed that can fully exploit the characteristics of the compounds. Therefore, there is much need for more effective treatments.

Patent Document 1 Japanese Translation of PCT International Application No. Hei 1-502746
Patent Document 2 United States Patent No. 5118666
Patent Document 3 United States Patent No. 5120712
Patent Document 4 United States Patent No. 5614492
Patent Document 5 Japanese Translation of PCT International Application No. Hei 10-500114
Patent Document 6 United States Patent Application Laid-Open No. 2002-0119146
Patent Document 7 United States Patent Application Laid-Open No. 2002-0160008
Patent Document 8 Japanese Patent Application Laid-Open No. Hei 7-2695
Patent Document 9 United States Patent Application Laid-Open No. 2003-0050237

Non-Patent Document 1 Tonyobyogaku, 1st ed., p64-74, 2002 Apr.25, SHINDAN TO CHIRYO SHA K.K.
Non-Patent Document 2 Nippon Rinsho, Special Ed., (Shin Jidai No Tonyobyogaku 3), NIPPON RINSYO SHA K.K. 422-8, 2002 Sept.28)
Non-Patent Document 3 Saishin Igaku, 2005, 60:36-42
Non-Patent Document 4 Diabetes, 2004, 53:2181-2189
Non-Patent Document 5 Curr. Opin. Investig. Drugs, 2004, 5:402-410
Non-Patent Document 6 Eur. J. Clin. Invest., 1997, 27:533-536
Non-Patent Document 7 Diabetes Care, 2003, 26:2929-2940
Non-Patent Document 8 J Physiol., 2004, 558:369-380
Non-Patent Document 9 J Clin. Invest., 1993, 91:301-307
Non-Patent Document 10 Endocrinology, 1995, 136:3585-3596
Non-Patent Document 11 Diabetes, 2002, 51:424-429
Non-Patent Document 12 Diabetes Care, 2004, 27:1335-1342
Non-Patent Document 13 Diabetes Care, 2004, 27:2628-2635

Non-Patent Document 14 Diabetes Care, 2002, 25:869-875
Non-Patent Document 15 Diabetes Care, 2004, 27:2874-2880
Non-Patent Document 16 Regul. Pept., 1999, 85:9-24
Non-Patent Document 17 Diabetologia, 2005, 48:616-620
Non-Patent Document 18 Diabetes, 2002, 51:2677-2682
Non-Patent Document 19 Diabetes, 2002, 51:943-950
Non-Patent Document 20 Proc. Natl. Acad. Sci., 2001, 98:12138-12143
Non-Patent Document 21 Diabetes, 1999, 48:1026-1034
Non-Patent Document 22 Pharmacology, 2005, 74:112-119
Non-Patent Document 23 Proc. Natl. Acad. Sci. USA, 1980, 80:5458-5489
Non-Patent Document 24 J. Clin. Invest., 1987, 79:616-619
Non-Patent Document 25 New England Medicine, 1992, 326:1316-1322
Non-Patent Document 26 Diabetes Care, 1992, 15:270-275

### DISCLOSURE OF THE INVENTION

### PROBLEMS TO BE SOLVED BY THE INVENTION

Accordingly, it is an object of the present invention to provide a novel dosage regimen of GLP-1 receptor agonists that causes little or no side effects or drug interactions and is suitable for improving insulin resistance. It is another object of the present invention to provide an insulin resistance improver for use in the dosage regimen.

Specifically, it is an object of the present invention to provide a method for improving insulin resistance without causing problems associated with the treatment of diabetes that uses existing insulin resistance improvers and biological activities of existing GLP-1 receptor agonists. It is another object of the present invention to provide a method for preventing or treating hyperglycemia, diabetes, obesity and other disorders that are associated with insulin resistance, as well as an insulin resistance improver for use in the method containing as an active ingredient a GLP-1 receptor agonist.

### MEANS FOR SOLVING THE PROBLEMS

The present inventors hypothesized that the biological activity of GLP-1 receptor agonists could be exploited more effectively and, thus, a novel clinical approach for the treatment of diabetes could be developed by administering a GLP-1 receptor agonist (i.e., GLP-1, a GLP-1 derivative or a pharmaceutically acceptable salt thereof) to patients with insulin resistance in a manner that creates a similar or enhanced circadian variation of the GLP-1 receptor agonist as compared to the circadian variation of the endogenous GLP-1 receptor agonist in a healthy individual. The present inventors conducted a study to prove this hypothesis.

The study has demonstrated that the best way to use GLP-1 receptor agonists in the treatment of diabetes is to administer, preferably by a non-invasive method, a GLP-1 receptor agonist to create a condition similar to what is observed with postprandial temporary secretion of the endogenous GLP-1 receptor agonist, rather than to administer it in such a manner that the GLP-1 receptor agonist is maintained at a high concentration for a prolonged period of time. The present inventors have found that this novel technique can safely and effectively ameliorate the insulin resistance in an individual.

One aspect of the present invention concerns the following:
(1) A pharmaceutical composition for improving insulin resistance, or for preventing or treating disorders associated with insulin resistance, the composition containing as an active ingredient a GLP-1 receptor agonist or a pharmaceutically acceptable salt thereof and intended for use on a continuous schedule involving administering the GLP-1 receptor agonist or a pharmaceutically acceptable salt thereof to an individual with insulin resistance in a single dose of 0.5 to 5000 pg at least once daily before eating for at least 2 weeks.
(2) The pharmaceutical composition according to (1) above, wherein the composition is also administered after eating.
(3) The pharmaceutical composition according to (1) or (2) above, wherein the GLP-1 receptor agonist is GLP-1(7-36) amide.
(4) The pharmaceutical composition according to any of (1) to (3) above, wherein the composition is administered either subcutaneously, nasally or pulmonarily.
(5) The pharmaceutical composition according to any of (1) to (4) above, wherein the disorder associated with insulin resistance is hyperinsulinemia, diabetes, obesity, hyperlipidemia, arteriosclerosis, hypertension or a cardiovascular disease.
(6) The pharmaceutical composition according to (7) above, wherein the disorder associated with insulin resistance is diabetes.
(7) The pharmaceutical composition according to (1) above, intended for nasally administering GLP-1(7-36) amide before eating.

Another aspect of the present invention concerns the following:
(8) A method for improving insulin resistance, or for preventing or treating disorders associated with insulin resistance, the method comprising a continuous schedule involving administering a GLP-1 receptor agonist or a pharmaceutically acceptable salt thereof to an individual with insulin resistance in a single dose of 0.5 to 5000 µg at least once daily before eating for at least 2 weeks.
(9) The method according to (8) above, wherein the GLP-1 receptor agonist is also administered after eating.
(10) The method according to (8) or (9) above, wherein the GLP-1 receptor agonist is GLP-1(7-36) amide.
(11) The method according to any of (8) to (10) above, wherein the GLP-1 receptor agonist is administered either subcutaneously, nasally or pulmonarily.
(12) The method according to any of (8) to (11) above, wherein the disorder associated with insulin resistance is hyperinsulinemia, diabetes, obesity, hyperlipidemia, arteriosclerosis, hypertension or a cardiovascular disease.
(13) The method according to (12) above, wherein the disorder associated with insulin resistance is diabetes.
(14) The method according to (8) above for nasally administering GLP-1(7-36) amide before eating.

Still another aspect of the present invention concerns the following:
(15) A use of GLP-1 receptor agonist in the production of a pharmaceutical composition for improving insulin resistance, or for preventing or treating disorders associated with insulin resistance, the composition containing as an active ingredient a GLP-1 receptor agonist or a pharmaceutically acceptable salt thereof and intended for use on a continuous schedule involving administering the GLP-1 receptor agonist or a pharmaceutically acceptable salt thereof to an individual with insulin resistance in a single dose of 0.5 to 5000 µg at least once daily before eating for at least 2 weeks.
(16) The use according to (15) above, wherein the composition is also administered after eating.
(17) The use according to (15) or (16) above, wherein the GLP-1 receptor agonist is GLP-1(7-36) amide.
(18) The use according to any of (15) to (17) above, wherein the GLP-1 receptor agonist is administered either subcutaneously, nasally or pulmonarily.
(19) The use according to any of (15) to (18) above, wherein the disorder associated with insulin resistance is hyperinsulinemia, diabetes, obesity, hyperlipidemia, arteriosclerosis, hypertension or a cardiovascular disease.
(20) The use according to (19) above, wherein the disorder associated with insulin resistance is diabetes.
(21) The use according to (15) above for nasally administering GLP-1(7-36) amide before eating.

Rather than continuously maintaining a high plasma concentration of GLP-1 receptor agonist, the invention described above intermittently or repeatedly causes a temporary increase in the plasma concentration of GLP-1 receptor agonist, thereby creating a similar circadian variation of GLP-1 receptor agonist as compared to the circadian variation of the endogenous GLP-1 receptor agonist. In this manner, the present invention can directly improve insulin resistance. The present inventors have for the first time discovered that such an approach is effective in ameliorating or treating hyperglycemia, diabetes, obesity and other disorders associated with insulin resistance.

This finding was made during a study in which intermittent or repeated temporary increases in the plasma concentration of a GLP-1 receptor agonist were induced by administering the GLP-1 receptor agonist to the overeating diabetic obese mouse model of insulin resistance (KK-Ay/Ta mice). While a single small dose of GLP-1 receptor agonist can hardly decrease the blood glucose levels due to the short half-life of the compound, the blood glucose level can be kept from increasing or even decreased by intermittently or repeatedly causing a temporary increase in the plasma concentration of the GLP-1 receptor agonist, rather than by continuously maintaining a high plasma concentration of the GLP-1 receptor agonist.

The decrease in the blood glucose levels resulting from the dosage regimen of the present invention does not necessarily require an increase in the insulin secretion. In fact, a comparison with the hypoglycemic effect of intraperitoneally administered insulin indicates that the dosage regimen of the present invention decreases the blood glucose levels through enhancement of the insulin sensitivity, or improvement of the insulin resistance. According to the dosage regimen of the present invention, the decrease in the blood glucose levels is observed sufficiently after the administration of the GLP-1 receptor agonist, when the exogenous GLP-1 receptor agonist has disappeared from the body. This observation suggests that the desired effect can be achieved without having to continuously maintain the high blood concentration of the GLP-1 receptor agonist as in the traditional approach.

To summarize, the insulin resistance in an individual can be improved by the dosage regimen of the present invention in which a GLP-1 receptor agonist is intermittently or repeatedly administered to cause intermittent, repeated temporary increases in the plasma concentration of the GLP-1 receptor agonist. As opposed to the traditional approach, the plasma concentration of the GLP-1 receptor agonist does not have to be continuously maintained high. As a result of the improvement of the insulin resistance, the hyperglycemia in the individual is improved.

Furthermore, studies have shown that continuously high blood insulin or glucose levels resulting from insulin resistance can cause hypertension, hyperlipidemia or cardiovascular disorders, the conditions collectively termed "metabolic syndrome" (Non-Patent Document 1). Thus, the insulin resistance improver of the present invention is expected to find applications not only in the treatment of type-2 diabetes, but also in the prevention and treatment of hyperlipidemia, hypertension, ischemic heart diseases and various other diseases.

According to the present invention, the improvement of insulin resistance is not a result of a secondary effect of the improved hyperglycemia or increased insulin secretion. For this reason, the insulin resistance improver of the present invention should be applicable not only to the treatment of patients with type-2 diabetes who suffer systemic insulin depletion, but also to the treatment of local decreases in the insulin sensitivity and associated diseases.

### EFFECT OF THE INVENTION

One feature of the present invention is that it has demonstrated that insulin resistance can be improved by repeatedly administering of GLP-1 receptor agonist for a certain period of time to cause intermittent temporary increases in the plasma concentration of the GLP-1 receptor agonist. The improved insulin resistance remains sufficiently after the administration of the GLP-1, when GLP-1 can no longer be detected in the blood. This indicates that the mechanism by which the present invention works is completely different from those previously reported for GLP-1 (such as stimulation of insulin secretion, enhancement of hypoglycemic activity of insulin in the presence of GLP-1 and insulin-independent glucose uptake).

In addition, GLP-1 does not cause liver function failure, edema, weight gain, an increase in adipose tissue weight and other side effects that are reported for the existing insulin resistance improvers. Thus, the insulin resistance improver of the present invention is suitable for use in obese patients or patients with cardiac failure and can offer more effective treatment than traditional insulin resistance improvers.

To summarize, the present invention offers the following advantages:
(1) When the insulin resistance improver is repeatedly administered for a predetermined period of time to insulin-resistant patients who still don't exhibit symptoms of hyperglycemic diabetes, the resulting intermittent, repeated and temporary increases in the plasma concentration of GLP-1 receptor agonist serve to prevent the onset of diabetes in the patients.
(2) When the insulin resistance improver is repeatedly administered for a predetermined period of time to diabetic patients who exhibit insulin resistance, the resulting intermittent, repeated and temporary increases in the plasma concentration of GLP-1 receptor agonist serve to improve the insulin resistance in the patients.
(3) When the insulin resistance improver is repeatedly administered for a predetermined period of time to diabetic patients who exhibit insulin resistance, the resulting intermittent, repeated and temporary increases in the plasma concentration of GLP-1 receptor agonist serve to improve the insulin resistance in the patients, thereby preventing pancreatic exhaustion and suppressing the progress of diabetes.
(4) When the insulin resistance improver is repeatedly administered for a predetermined period of time to patients who exhibit symptoms of insulin resistance but do not necessarily exhibit increased blood glucose levels, the resulting intermittent, repeated and temporary increases in the plasma concentration of GLP-1 receptor agonist serve to improve the insulin resistance in the patients.

### BRIEF DESCRIPTION OF THE DRAWINGS

Fig. 1 shows the changes in the postprandial blood glucose levels of KK-Ay/TaJcl mice repeatedly administered GLP-1(7-36) amide (500 µg/kg, s.c.) or troglitazone (30 mg/kg, i.p.) once daily.
Fig. 2 shows a comparison in the fasting blood glucose levels of KK-Ay/TaJcl mice repeatedly administered GLP-1(7-36) amide (500 µg/kg, s.c.) or troglitazone (30 mg/kg, i.p.) once daily for 13 days.
Fig. 3 shows the serum radioactivity of KK-Ay/TaJcl mice repeatedly administered GLP-1(7-36) amide (500 µg/kg, s.c.) or troglitazone (30 mg/kg, i.p.) once daily for 14 days and subsequently orally fed a mixed solution of glucose/³H-deoxyglucose (measurements taken 30 minutes after the glucose loading).
Fig. 4 shows the serum glucose levels of KK-Ay/TaJcl mice repeatedly administered GLP-1(7-36) amide (500 µg/kg, s.c.) or troglitazone (30 mg/kg, i.p.) once daily for 14 days and subsequently orally fed a mixed solution of glucose/ ³H- deoxyglucose (measurements taken 30 minutes after the glucose loading).

Fig. 5 shows the amount of the radioactivity transferred to different tissues in KK-Ay/TaJcl mice repeatedly administered GLP-1(7-36) amide (500 µg/kg, s.c.) or troglitazone (30 mg/kg, i.p.) once daily for 14 days and subsequently orally fed a mixed solution of glucose/³H-deoxyglucose (measurements taken 30 minutes after the glucose loading).
Fig. 6 shows the changes in the plasma concentration of active GLP-1 in mice subcutaneously administered a GLP-1(7-36) amide solution (medium: 25% dextran 70 solution, 500 µg/kg).
Fig. 7 shows the baseline blood glucose levels (measured at 9 o'clock) observed during a period in which GLP-1(7-36) amide (15, 50 or 150 µg/kg) was subcutaneously administered 3 times daily.
Fig. 8 shows the baseline blood glucose levels (measured at 17 o'clock) observed during a period in which GLP-1(7-36) amide (15, 50 or 150 µ*g/kg) was subcutaneously administered 3 times daily.

Fig. 9 shows the changes in the glycosylated hemoglobin levels (HbA1c, measured at 9 o'clock) observed during a period in which GLP-1(7-36) amide (15, 50 or 150 µg/kg) was subcutaneously administered 3 times daily.
Fig. 10 shows the changes in the serum insulin levels (measured at 9 o'clock) observed during a period in which GLP-1(7-36) amide (15, 50 or 150 µg/kg) was subcutaneously administered 3 times daily.

Fig. 11 shows the decreases in the blood glucose levels after subcutaneous administration of insulin (0.75 U/kg) in KK-Ay/TaJcl mice subcutaneously and repeatedly administered GLP-1(7-36) amide (15, 50 or 150 µg/kg) 3 times daily over an 8 week-period.
Fig. 12 shows the changes in the plasma concentration of active GLP-1 in mice subcutaneously administered a GLP-1(7-36) amide solution (medium: physiological saline, 50 µg/kg).
Fig. 13 shows the changes in the plasma concentration of active GLP-1 in cynomolgus monkeys nasally administered 30 mg of a powder preparation containing 30 µg or 100 pg GLP-1(7-36) amide.

Fig. 14.1 shows the changes in body weight in KK/TaJcl mice fed a normal diet or high fat diet, observed during a 15-week period in which GLP-1(7-36) amide was subcutaneously administered 3 times daily.
Fig. 14.2 shows the changes in the amount of food intake in KK/TaJcl mice fed a normal diet or high fat diet, observed during a 15-week period in which GLP-1(7-36) amide was subcutaneously administered 3 times daily.
Fig. 14.3 shows the changes in the random blood glucose levels in KK/TaJcl mice fed a normal diet or high fat diet, observed during a 15-week period in which GLP-1(7-36) amide was subcutaneously administered 3 times daily (measurements taken each day before administration at 9 o'clock).
Fig. 14.4 shows the changes in the glycosylated hemoglobin levels (HbA1c) in KK/TaJcl mice fed a normal diet or high fat diet, observed during a 15-week period in which GLP-1(7-36) amide was subcutaneously administered 3 times daily.
Fig. 14.5 shows the changes in the plasma insulin levels in KK/TaJcl mice fed a normal diet or high fat diet, observed during a 15-week period in which GLP-1(7-36) amide was subcutaneously administered 3 times daily (measurements taken each day before administration at 9 o'clock).

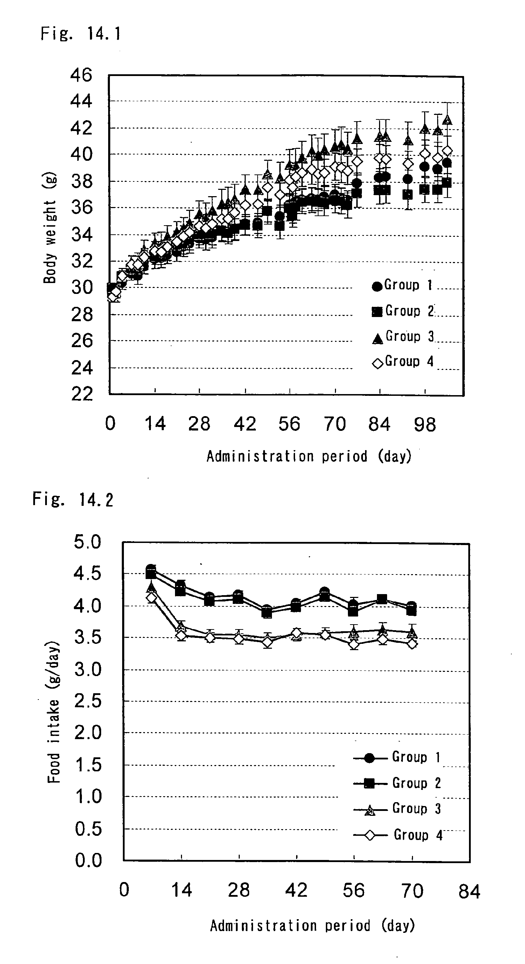

In Fig. 14.1 through Fig. 14.5, Group 1 is fed a normal diet (medium), Group 2 a normal diet (GLP-1(7-36) amide), Group 3 a high fat diet (medium), and Group 4 a high fat diet (GLP-1(7-36) amide). For the comparison of Group 1 and Group 3, *P < 0.05 and **p < 0.01; for the comparison of Group 3 and Group 4, #P < 0.05 and ##p < 0.01.

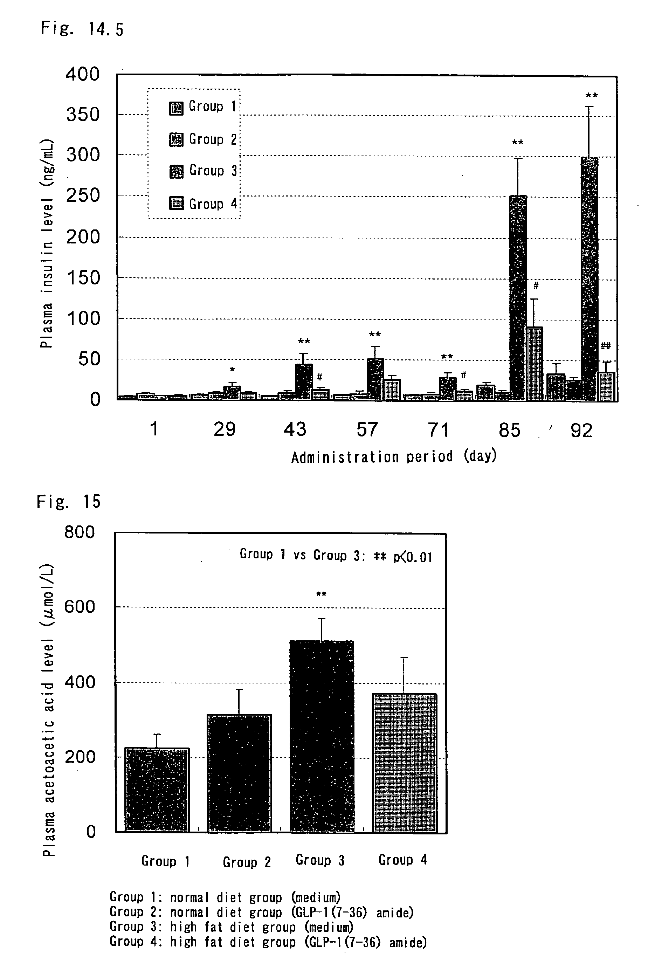

Fig. 15 is a diagram showing the concentration of acetoacetic acid in KK/TaJcl mice fed a normal diet or high fat diet, observed during a 15-week period in which GLP-1(7-36) amide was subcutaneously administered 3 times daily.

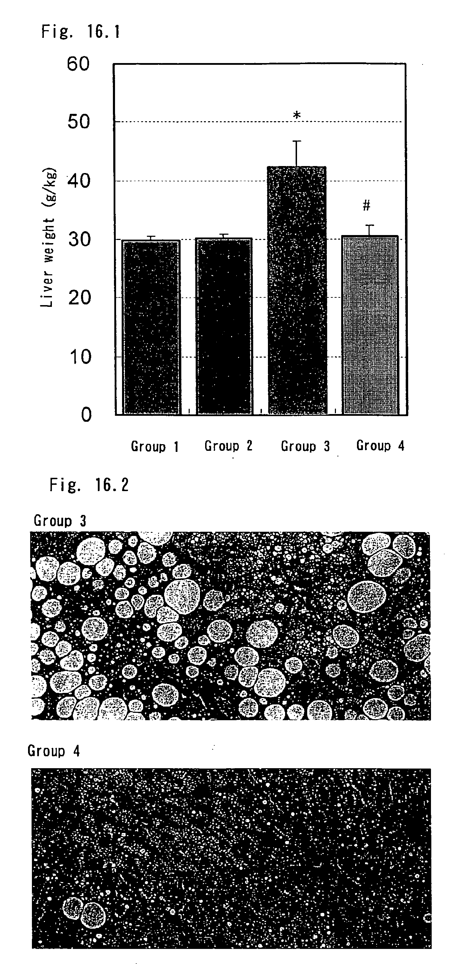

Fig. 16.1 is a diagram showing the weight of the liver collected from KK/TaJcl mice fed a normal diet or high fat diet and repeatedly administered GLP-1(7-36) amide 3 times daily over a 22-to 24-week period.
Fig. 16.2 shows micrographs of liver tissue samples collected from KK/TaJcl mice fed a high fat diet and repeatedly administered GLP-1(7-36) amide 3 times daily over a 22- to 24-week period.
In Fig. 16.1 and Fig. 16.2, Group 1 is fed a normal diet (medium), Group 2 a normal diet (GLP-1(7-36) amide), Group 3 a high fat diet (medium), and Group 4 a high fat diet (GLP-1(7-36) amide). For the comparison of Group 1 and Group 3, *P < 0.05; for the comparison of Group 3 and Group 4, #P < 0.05.

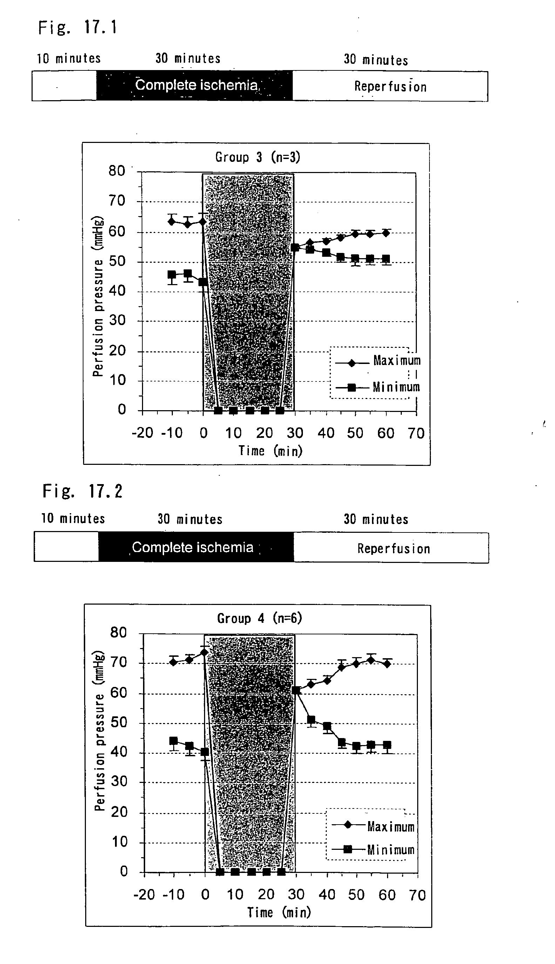

Fig. 17.1 is a diagram showing the results of an evaluation of cardiac function by Langendorff's perfusion technique, performed on hearts excised from animals fed a high fat diet and repeatedly administered the medium over a 22- to 24-week period (Group 3).
Fig. 17.2 is a diagram showing the results of an evaluation of cardiac function by Langendorff's perfusion technique, performed on hearts excised from animals fed a high fat diet and repeatedly administered GLP-1(7-36) amide over a 22- to 24-week period (Group 4).

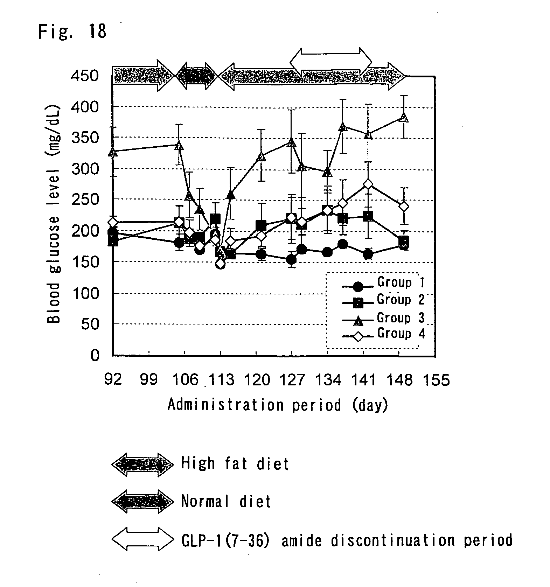

Fig. 18 is a diagram showing the changes in the blood glucose levels in ICR mice repeatedly administered GLP-1(7-36) amide 3 times daily over a 15-week period and subsequently switched to a different diet, as well as changes in the blood glucose levels upon termination of GLP-1.

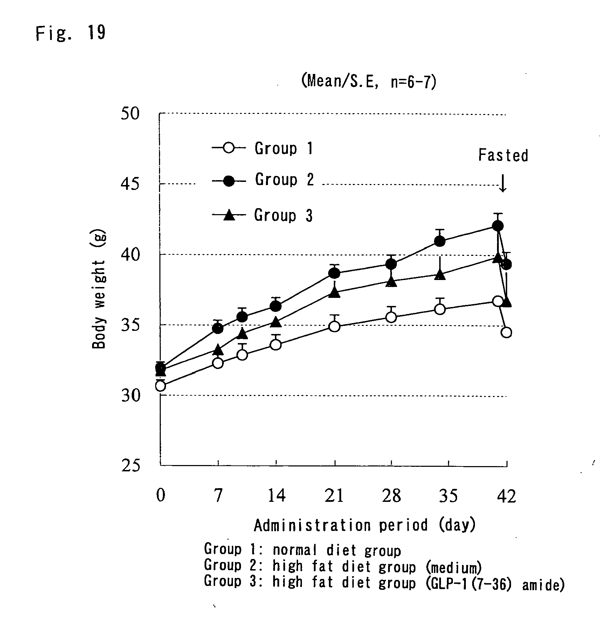

Fig. 19 is a diagram showing the changes in body weight for the control group and the GLP-1(7-36) amide group of CRj:CD1(ICR) mice kept on a normal diet or high fat diet.

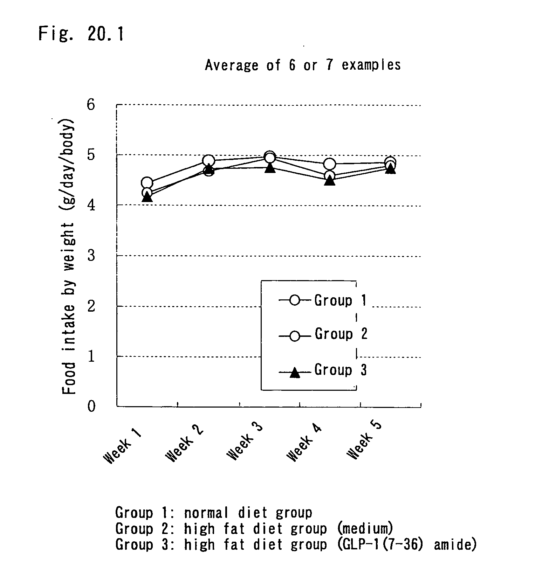

Fig. 20.1 is a diagram showing the changes in the weight of food intake for the control group and the GLP-1 group of CRj:CD1(ICR) mice kept on a normal diet or high fat diet (one group was kept in a cage and the average food intake was determined by dividing the total food consumption of the group by the number of animals).

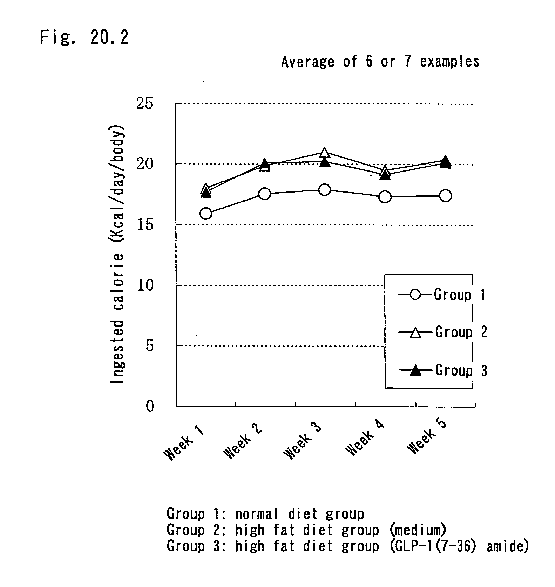

Fig. 20.2 is a diagram showing the changes in the calorie intake for the control group and the GLP-1(7-36) amide group of CRj:CD1(ICR) mice kept on a normal diet or high fat diet (calorie intake was determined as the weight of food intake multiplied by the number of calories per unit weight of the diet).

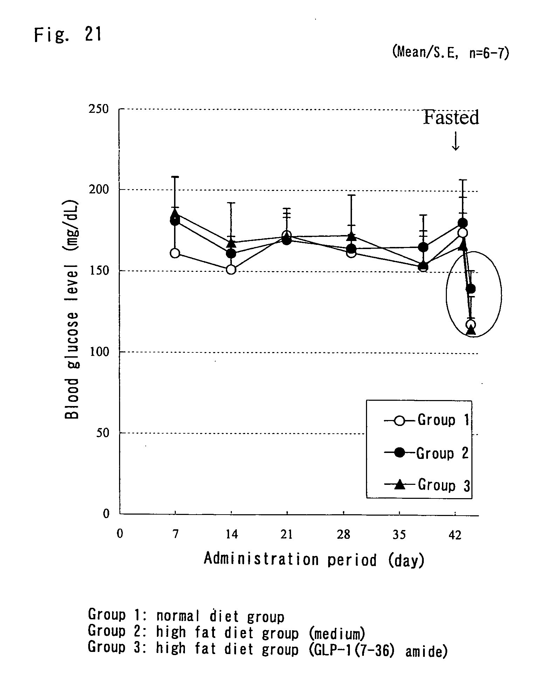

Fig. 21 is a diagram showing the changes in the blood glucose levels during the administration period for the control group and the GLP-1(7-36) amide group of CRj:CD1(ICR) mice kept on a normal diet or high fat diet.

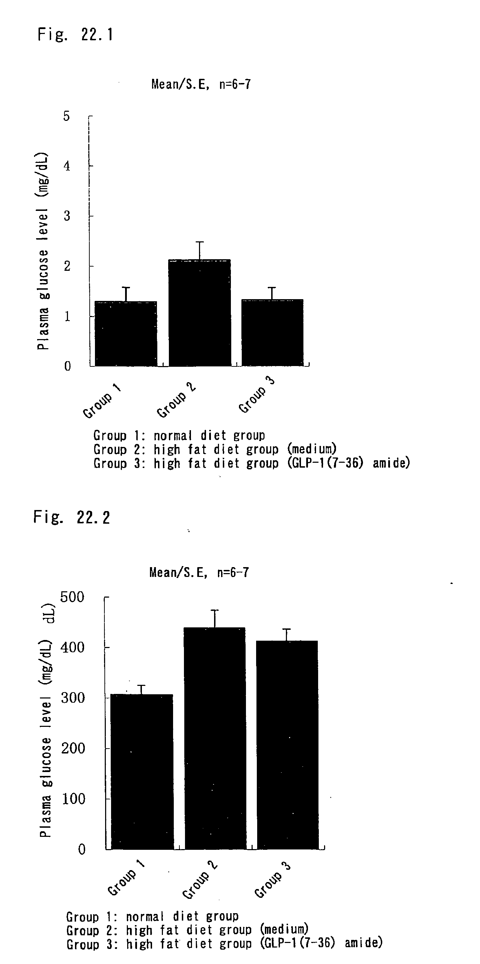

Fig. 22.1 is a diagram showing the plasma glucose levels measured 30 minutes after the subcutaneous administration of 1.5 g/kg glucose to CRj:CD1(ICR) mice that had been repeatedly administered GLP-1(7-36) amide or medium once or twice daily over the preceding 5-week period and fasted for one night after the final administration.
Fig. 22.2 is a diagram showing the insulin levels measured 30 minutes after the subcutaneous administration of 1.5 g/kg glucose to CRj:CD1(ICR) mice that had been repeatedly administered GLP-1(7-36) amide or medium once or twice daily over the preceding 5-week period and fasted for one night after the final administration.

### BEST MODE FOR CARRYING OUT THE INVENTION

As used herein, the term "GLP-1 receptor agonist" refers to GLP-1 (Non-Patent Document 23, SEQ ID: NO. 1) or a GLP-1 derivative that binds to the GLP-1 receptor to stimulate insulin secretion.

GLP-1 is a 37-amino acid peptide derived from preproglucagon. Preproglucagon is also processed to form GLP-1(7-37), in which the 6 amino acids at the N-terminal of GLP-1 have been deleted, or GLP-1(7-36) amide, in which the C-terminal of GLP-1(7-36) has been modified to form an amide (Non-Patent Document 24). Since these peptide hormones (i.e., GLP-1 and GLP-1 derivatives) act on the pancreatic beta cells and stimulate insulin secretion, their possibility as a treatment for diabetes has been suggested (Non-Patent Documents 25 and 26).

In addition to GLP-1(7-37) and GLP-1(7-36) amide described above, GLP-1 derivatives include other peptides that act to stimulate insulin secretion and are formed by substitution, addition or deletion of one or more amino acid residues from GLP-1, the 37-amino acid peptide, as well as the peptides formed by modification of amino acids in these peptides (e.g., amides) and combinations thereof that act to stimulate insulin secretion.

In addition to those described in examples of the present invention, specific examples of the GLP-1 derivative include the following:
(1) GLP-1(7-34), GLP-1(7-35), GLP-1(7-36), GLP-1(7-34)amide, GLP-1(7-35)amide and GLP-1(7-37)amide;
(2) GLP-1(7-37)-Arg, GLP-1(7-37)-Arg-Arg, GLP-1(7-37)-Lys, GLP-1(7-37)-Lys-Lys, GLP-1(7-37)-Lys-Arg and GLP-1(7-37)-Arg-Lys, and C-terminal amides thereof;
(3) GLP-1(7-37) obtained by substitution of Ala at position 8 of GLP-1 for Thr, Gly or Ser, and GLP-1(7-36) amides;
(4) GLP-1(7-37) obtained by substitution of Lys at position 26 of GLP-1 for Arg, and GLP-1(7-36) amides;
(5) GLP-1(7-37) obtained by substitution of Lys at position 34 of GLP-1 for Arg, and GLP-1(7-36) amides;
(6) GLP-1(7-37) obtained by substitution of Arg at position 36 of GLP-1 for Lys, and GLP-1(7-36) amides;
(7) GLP-1(7-37) obtained by substitution of Ala at position 8 of GLP-1 for Thr, Gly or Ser and substitution of Lys at position 26 for Arg, and GLP-1(7-36) amides;
(8) GLP-1(7-37) obtained by substitution of Ala at position 8 of GLP-1 for Thr, Gly or Ser and substitution of Lys at position 34 for Arg, and GLP-1(7-36) amides;
(9) GLP-1(7-37) obtained by substitution of Ala at position 8 of GLP-1 for Thr, Gly or Ser and substitution of Arg at position 36 for Lys, and GLP-1(7-36) amides;
(10) GLP-1(7-37) obtained by substitution of Lys at position 26 of GLP-1 for Arg and substitution of Lys at position 34 for Arg, and GLP-1(7-36) amides;
(11) GLP-1(7-37) obtained by substitution of Lys at position 26 of GLP-1 for Arg and substitution of Arg at position 36 for Lys, and GLP-1(7-36) amides;
(12) GLP-1(7-37) obtained by substitution of Lys at position 34 of GLP-1 for Arg and substitution of Arg at position 36 for Lys, and GLP-1(7-36) amides;
(13) GLP-1(7-37) obtained by substitution of Ala at position 8 of GLP-1 for Thr, Gly or Ser, substitution of Lys at position 26 for Arg and substitution of Lys at position 34 for Arg, and GLP-1(7-36) amides;
(14) GLP-1(7-37) obtained by substitution of Ala at position 8 of GLP-1 for Thr, Gly or Ser, substitution of Lys at position 26 for Arg and substitution of Arg at position 36 for Lys, and GLP-1(7-36) amides;
(15) GLP-1(7-37) obtained by substitution of Ala at position 8 of GLP-1 for Thr, Gly or Ser, substitution of Lys at position 34 for Arg and substitution of Arg at position 36 for Lys, and GLP-1(7-36) amides;
(16) GLP-1(7-37) obtained by substitution of Lys at position 26 of GLP-1 for Arg, substitution of Lys at position 34 for Arg and substitution of Arg at position 36 for Lys, and GLP-1(7-36) amides;
(17) GLP-1(7-37) obtained by substitution of Ala at position 8 of GLP-1 for Thr, Gly or Ser, substitution of Lys at position 26 for Arg, substitution of Lys at position 34 for Arg and substitution of Arg at position 36 for Lys, and GLP-1(7-36) amides;

GLP-1 receptor agonists suitable for use in the present invention are GLP-1 derivatives such as GLP-1(7-37) and GLP-1(7-36) amides. GLP-1(7-36) amides are particularly preferred.

The GLP-1 receptor agonists for use in the present invention may be obtained by known techniques, such as chemical synthesis, genetic recombination and combinations thereof.

The GLP-1 receptor agonists for use in the present invention are preferably provided in the form of pharmaceutically acceptable salts, such as salts formed with inorganic bases, organic bases, inorganic acids, organic acids, or basic or acidic amino acids.

Preferred examples of the salts formed with an inorganic base include alkali metal salts, such as sodium salts and potassium salts, alkaline earth metal salts, such as calcium salts and magnesium salts, aluminum salts, and ammonium salts.

Preferred examples of the salts formed with an organic base include salts formed with trimethylamine, triethylamine, pyridine, picoline, ethanolamine, diethanolamine, triethanolamine, dicyclohexylamine or N,N'-dibenzylethylenediamine.
Preferred examples of the salts formed with an inorganic acid include salts formed with hydrochloric acid, hydrobromic acid, nitric acid, sulfuric acid or phosphoric acid.

Preferred examples of the salts formed with an organic acid include salts formed with formic acid, acetic acid, trifluoroacetic acid, fumaric acid, oxalic acid, tartaric acid, maleic acid, citric acid, succinic acid, malic acid, methanesulfonic acid, benzenesulfonic acid or p-toluenesulfonic acid.

Preferred examples of the salts formed with a basic amino acid include salts formed with arginine, lysine or ornithine. Preferred examples of the salts formed with an acidic amino acid include salts formed with aspartic acid or glutamic acid.
Of these salts, sodium salts and potassium salts are most preferred.

The GLP-1 receptor agonist or a pharmaceutically acceptable salt thereof may be mixed with a pharmaceutically acceptable carrier, excipient or expander to form a pharmaceutical preparation containing the GLP-1 receptor agonist or a pharmaceutically acceptable salt thereof as an active ingredient. Such a pharmaceutical preparation can be used in individuals including humans, mice, rats, rabbits, canines, felines, bovines, equines, porcines and monkeys.

Although the GLP-1 receptor agonist of the present invention may be administered at any suitable dose determined depending on age, body weight and species of the individual and the purpose of the pharmaceutical composition, it is preferably administered at a dose that causes an increase in the plasma levels of GLP-1 receptor agonist in a healthy individual that corresponds to the increase in the endogenous GLP-1 receptor agonist levels induced after eating in the individual. Specifically, it is administered at a dose effective in temporarily bringing the plasma level of GLP-1 receptor agonist (which is also referred to as "active GLP-1" in the present application) in an individual to a concentration of 20 pg/ml or higher.
As used herein, the term "temporarily" means that the plasma level of the GLP-1 receptor agonist is maintained at 20p g/ml or higher for about 1 to 3 hours after the administration.

The amount of the GLP-1 receptor agonist for administration is adjusted so that the preparation can preferably deliver 0.5 pg to 5000 pg of the GLP-1 receptor agonist, more preferably 5.0 pg to 5000 pg of the GLP-1 receptor agonist, in a single dose. The pharmaceutical preparation formulated in this manner is administered on a continuous schedule of at least once daily for at least 2 weeks.

For example, when GLP-1(7-36) amide is nasally administered to an adult human as a GLP-1 receptor agonist, the amount of GLP-1(7-36) amide in the preparation is preferably adjusted to 300 µg to 5000 pg. The dose is determined as the amount required to establish, in a human subject, the same plasma level as that proved effective in improving insulin resistance in mice in Examples 1 and 2. Specifically, the dose of 300 pg is expected to raise the plasma level of GLP-1 receptor agonist to substantially the same level as is achieved in mice subcutaneously administered 15 µg/kg of GLP-1(7-36) amide. Likewise, the dose of 5000 pg is expected to raise the plasma level of GLP-1 receptor agonist to substantially the same level as is achieved in mice subcutaneously administered 500 µg/kg of GLP-1(7-36) amide.

The preparation is administered on a continuous schedule of at least once daily, before each meal, for at least 2 weeks.

The preparation may be administered not only before eating, but also after eating. For human subjects, it is preferably administered before or after each meal, for example, three times daily.
Preferably, the interval between each administration is adjusted such that the plasma level of the GLP-1 receptor agonist (active GLP-1) remains as low as 20 pg/mL or lower for more than 1 hour.

A safety evaluation test using the same type of preparation as the powder nasal GLP-1(7-36) amide preparation used in Example 3 revealed that the nasal preparation did not show any toxicity changes or cause irritancy to nasal mucosa in cynomolgus monkeys after a two-week period of treatment during which 3600 pg GLP-1(7-36) amide was administered 6 times daily.

The GLP-1 receptor agonist or a pharmaceutically acceptable salt thereof may be formulated with a pharmaceutically acceptable carrier to form solid preparations, such as tablets, capsules, granules and powders, or liquid preparations, such as syrups and injections for oral or parenteral administration.

The pharmaceutically acceptable carrier may be any organic or inorganic carrier material commonly used in the production of pharmaceutical preparations. For example, solid preparations may be formulated with an excipient, a lubricant, a binder or a disintegrating agent. Liquid preparations may be formulated with a solvent, a solubilizing agent, a suspending agent, an isotonizing agent, a buffer or a pain reliever.
When necessary, additives such as antiseptics, antioxidants, coloring agents and sweeteners may be added.

Preferred examples of the excipient include lactose, sucrose, D-mannitol, starch, crystalline cellulose and light silicic anhydride. Preferred examples of the lubricant include magnesium stearate, calcium stearate, talc and colloidal silica.

Preferred examples of the binder include crystalline cellulose, sucrose, D-mannitol, dextrin, hydroxypropylcellulose, hydroxypropylmethylcellulose and polyvinylpyrrolidone.

Preferred examples of the disintegrating agent include starch, carboxymethylcellulose, carboxymethylcellulose calcium, croscarmellose sodium and carboxymethyl starch sodium.

Preferred examples of the solvent include water for injection, alcohols, propylene glycol, macrogol, sesame oil and corn oil.

Preferred examples of the solubilizing agent include polyethylene glycol, propylene glycol, D-mannitol, benzyl benzoate, ethanol, trisaminomethane, cholesterol, triethanolamine, sodium carbonate and sodium citrate.

Preferred examples of the suspending agent include surfactants, such as stearyl triethanolamine, sodium lauryl sulfate, laurylaminopropionic acid, lecithin, benzalkonium chloride, benzethonium chloride and glyceryl monostearate, and hydrophilic polymers, such as polyvinyl alcohol, polyvinylpyrrolidone, carboxymethylcellulose sodium, methylcellulose, hydroxymethylcellulose, hydroxyethylcellulose and hydroxypropylcellulose.

Preferred examples of the isotonizing agent include sodium chloride, glycerin and D-mannitol.

Preferred examples of the buffer include phosphate buffer, acetate buffer, carbonate buffer and citrate buffer.

Preferred examples of the pain reliever include benzyl alcohol.
Preferred examples of the antiseptic include paraoxybenzoic acid esters, chlorobutanol, benzyl alcohol, phenethyl alcohol, dehydroacetic acid and sorbic acid.
Preferred examples of the antioxidant include sulfites and ascorbic acid.

Examples of the dosage form suitable for parenteral administration include nasal drops, injections, infusions, suppositories, transdermal preparations, transmucosal preparations and inhalants for nasal, pulmonary, intravenous, intracutaneous, subcutaneous or intramuscular administration. Examples of the dosage form suitable for oral administration include capsules, tablets and syrups. Of these dosage forms, parenteral preparations such as nasal preparations, injections, infusions, transmucosal preparations and inhalants are particularly suitable for use in the present invention.

These dosage forms are known by those skilled in the art: A person skilled in the art will be able to choose a dosage form suitable for the desired route of administration and prepare a pharmaceutical preparation in the dosage form by using, if necessary, one or more pharmaceutical additives known in the art.

For example, a pharmaceutical preparation in the form of an injection or infusion can be prepared by dissolving, along with a GLP-1 receptor agonist, the active ingredient, one or more of pharmaceutical additives, such as isotonizing agents, pH conditioners, pain relievers and antiseptics, in a distilled water for injection and sterilizing the solution. The pharmaceutical preparation in the form of an injection or infusion may be provided in the form of a freeze-dried product. Such a preparation can be prepared as an injection or infusion by dissolving in distilled water for injection or a physiological saline upon use. Preparations suitable for transmucosal administration include a nasal drop, nasal spray and other nasal preparations, as well as oral preparations, such as sublingual preparations, and pulmonary inhalants.

While the insulin resistance improver of the present invention may be administered via any suitable route of administration, it is preferably administered parenterally, via nasal, pulmonary, intravenous, intracutaneous or subcutaneous administration. The route of administration is preferably a non-invasive route, such as nasal or pulmonary administration.

### Examples

The present invention will now be described with reference to

### examples.

### Example 1: Activity of GLP-1(7-36) amide subcutaneously and repeatedly administered to KK-Ay/Ta Jcl insulin resistance mouse model over a two-week period

GLP-1(7-36) amide was dissolved in a 25% dextran 70 solution. The solution was subcutaneously administered at a dose of 500 µg/kg/day to male KK-Ay/Ta Jcl mice once daily for 14 consecutive days. The blood glucose levels and the glucose uptake by different tissues were monitored during the administration period. The animals were kept under a 12h light/12h dark circadian cycle (light phase: 7 - 19 o'clock) and fed a normal diet (solid diet (CRF-1), Oriental Yeast Co., Ltd.) during the repeated administration period.

### (1) Groups

Animals were divided into three groups: a drug-free control group (N = 4), a test compound group (N = 4) subcutaneously administered 500 µg/kg/day of GLP-1(7-36) amide once daily in the evening (at around 16 o'clock), and a positive control group (N = 4) intraperitoneally administered 30 mg/kg of troglitazone, a traditional insulin resistance improver, once daily in the evening (at around 16 o'clock).

### (2) Evaluation and Results

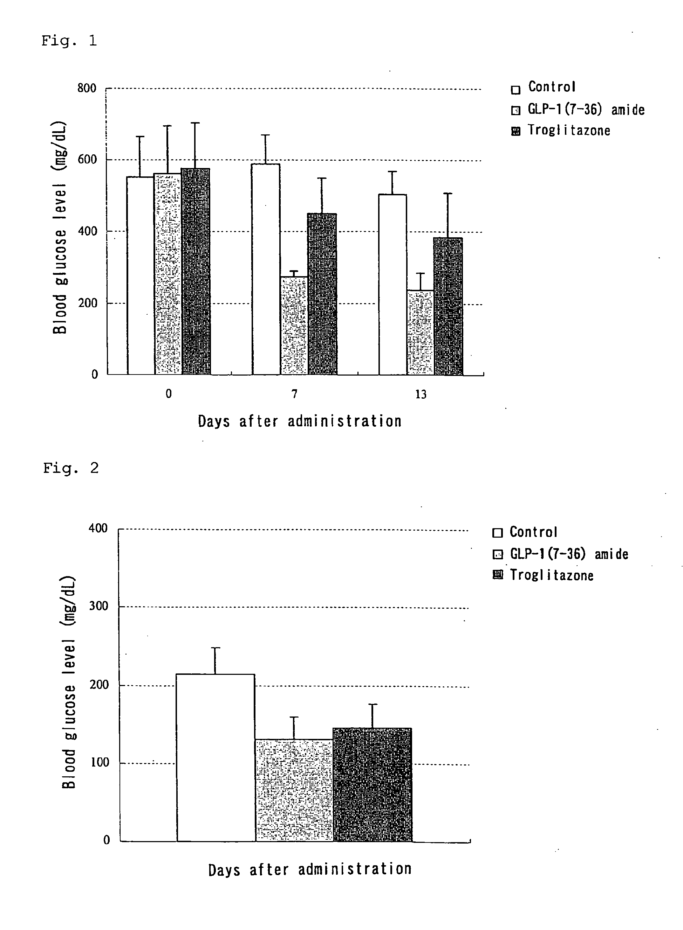

(a) Using a portable blood glucose meter (ANTOSENSE II, Bayer-Sankyo), the blood glucose levels (postprandial blood glucose levels before drug administration) were measured before administration of the test compound or the positive control compound, and on Day 7 and Day 13 of the administration period. The results are shown in Fig. 1.
   The GLP-1(7-36) amide group showed a greater decrease in the postprandial blood glucose levels than the control group during the administration period. The effect in the GLP-1(7-36) amide group was more significant than in the troglitazone group.
(b) The animals were repeatedly administered the test compound or the positive control compound for 13 consecutive days and were subsequently fasted for about 20 hours. Using a portable blood glucose meter (ANTOSENSE II, Bayer-Sankyo), the fasting blood glucose levels were measured. The results are shown in Fig. 2.
   The GLP-1(7-36) amide group showed a significant decrease in the fasting blood glucose levels. The effect was comparable to troglitazone.

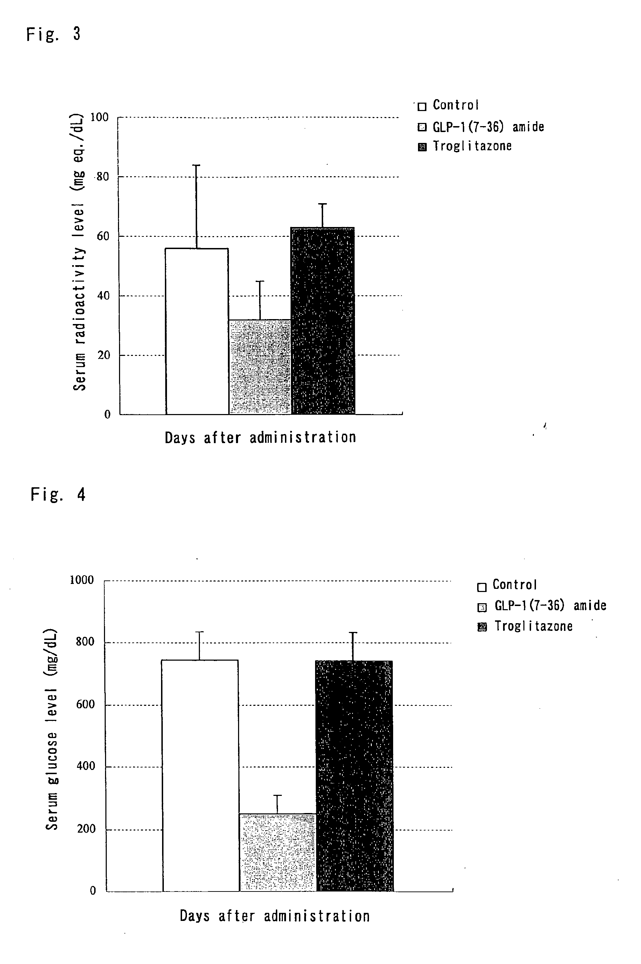

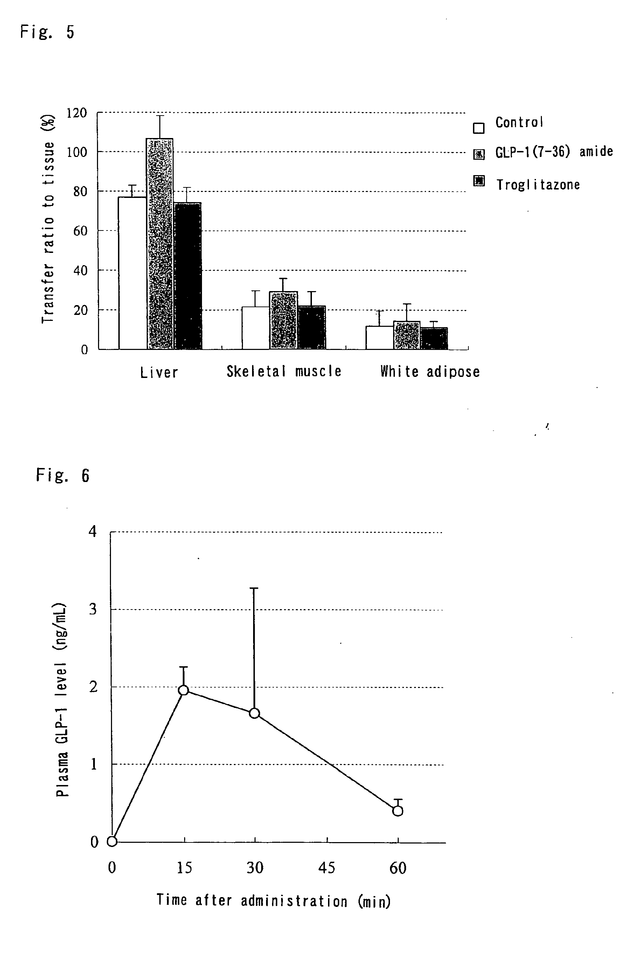

(c) The animals were repeatedly administered the test compound or the positive control compound for 14 consecutive days. 30 minutes after the final administration, a mixed solution of 0.15 g/mL glucose - 0.123 µg/0.83MBq/mL ³H-deoxyglucose (10 mL/kg) was orally administered. The animals were fasted for about 20 hours following the administration on Day 13. 30 minutes after the glucose load, the serum radioactivity levels and the serum glucose levels were measured by using a liquid scintillation counter (TRICARB 2200, Packard) and a mutase/GOD technique, respectively. The results are shown in Figs. 3 and 4.
Using a liquid scintillation counter (TRICARB 2200, Packard), the radioactivity level in liver, white adipose and skeletal muscles were also measured. The results are shown in Fig. 5.
The GLP-1(7-36) amide group showed a greater decrease in the serum radioactivity and the serum glucose levels and a greater increase in the uptake of radioactivity by liver as compared to the control group. No significant difference was observed in any of the measurements between the troglitazone group and the control group.

(d) GLP-1(7-36) amide was dissolved in a 25% dextran 70 solution and the resulting solution was subcutaneously administered to healthy mice (C57BL mice) at a dose of 500 µg/kg. The changes in the plasma levels of active GLP-1 were monitored (Fig. 6).
As shown, the plasma level of active GLP-1 increased to approximately 2 ng/mL 15 minutes after the administration and decreased to 0.4 ng/mL 1 hour after the administration.

### (3) Conclusion

KK-Ay/Ta Jcl mice are a mouse model of type-2 diabetes in which hyperinsulinemia and hyperglycemia are induced by overfeeding and insulin resistance. This line of animals does not respond to sulfonylurea agent, a hypoglycemic agent.
KK-Ay/Ta Jcl mice repeatedly administered 500 µg/kg of GLP-1(7-36) amide once daily for 13 consecutive days showed a significant decrease in the postprandial and fasting blood glucose levels during the administration period. This observation suggests that GLP-1(7-36) amide has an ability to improve insulin resistance. A mixed solution of glucose/³H-deoxyglucose orally given 30 minutes after the administration on Day 14 caused a decrease both in the serum glucose level and in the serum radioactivity level and caused an increase in the transfer of the radioactivity to liver and muscles.

In this example, the increased glucose uptake by the peripheral tissue was observed in the presence of GLP-1(7-36) amide administered immediately before. We speculated that, once insulin resistance has been improved by the action of the GLP-1 receptor agonist, the enhancement of the glucose uptake by the peripheral tissue must be observed not only immediately after the administration, but also sufficiently long after the administration when little or no active GLP-1 is present in the plasma. We conducted the following experiments to prove this assumption.

### Example 2: Activity of GLP-1(7-36) amide subcutaneously and repeatedly administered to KK-Ay/Ta Jcl insulin resistance mouse model over a 8-week period

GLP-1(7-36) amide was dissolved in physiological saline. The solution was subcutaneously administered to male KK-Ay/Ta Jcl mice three times daily for 8 weeks. The effects on the baseline blood glucose levels, glycosylated hemoglobin (HbA1c) levels and plasma insulin levels were examined. Following the final administration, the animals were intraperitoneally administered 0.75 U/kg of insulin and the blood glucose levels were measured over time. The animals were kept under a 12h light/12h dark circadian cycle (light phase: 8 - 20 o'clock) and fed a normal diet (solid diet (CRF-1), Oriental Yeast Co., Ltd.) during the repeated administration period.

### (1) Groups

Animals were divided into four groups: a control group (N = 10) administered the medium (physiological saline), and three test compound groups subcutaneously administered 15, 50 and 150 µg/kg of GLP-1(7-36) amide 3 times daily (at around 9, 13 and 17 o'clock) for 8 weeks.

### (2) Evaluation and Results

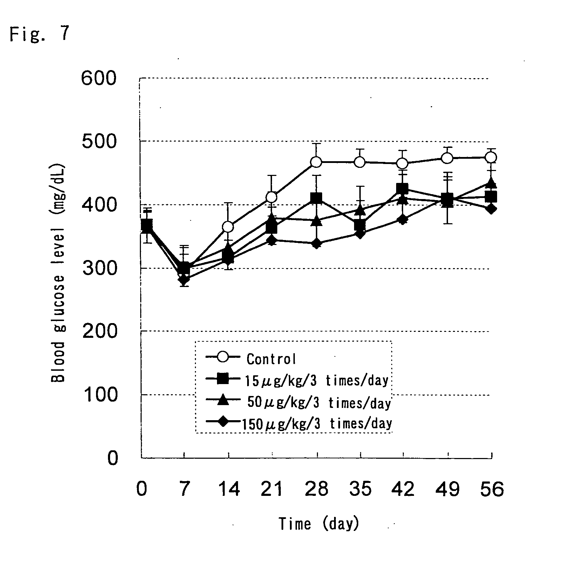

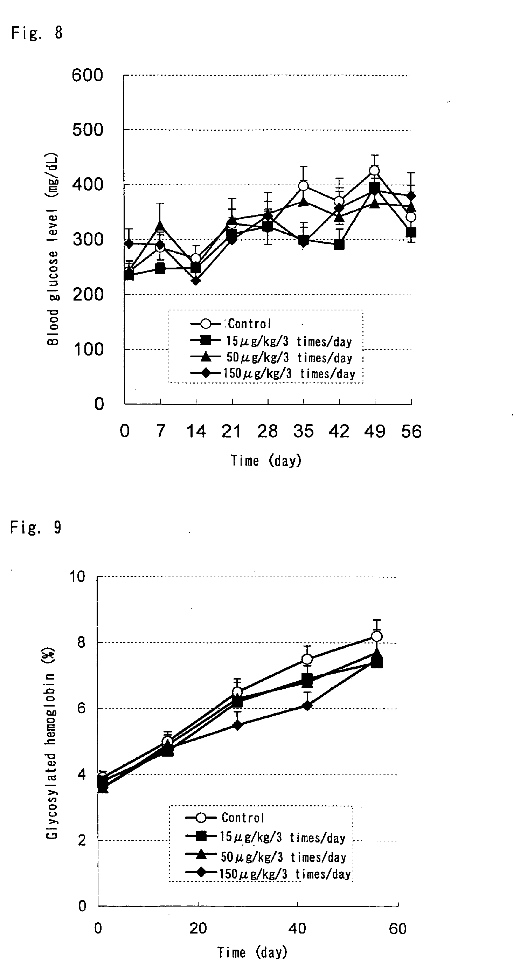

(a) Animals were subcutaneously administered GLP-1(7-36) amide 3 times daily (at around 9, 13 and 17 o'clock) for 8 weeks. The blood glucose levels before administration (at 9 and 17 o'clock), the glycosylated hemoglobin (HbA1c) levels (at 9 o'clock) and the plasma insulin levels before administration (at 9 o'clock) were measured throughout the administration period. The results are shown in Figs. 7 through 10.
As shown, the increase in the blood glucose levels (at 9 o'clock) and HbA1c (at 9 o'clock) levels was slightly suppressed during the administration period in each of the test compound groups. No significant difference was observed in the blood glucose levels measured before administration at 17 o'clock (9 o'clock). The increase in the insulin levels (at 9 o'clock) was suppressed during the administration period at doses of 15 to 150 µg/kg. Moreover, after the 8-week period of repeated subcutaneous administration, the hypoglycemic effect upon the intraperitoneal administration of insulin (0.75 U/kg) was enhanced although the fasting blood glucose levels following a fasting period of more than 16 hours were substantially the same in all groups. Since about 16 hours had passed since the previous administration by 9 o'clock when the measurements were taken for the blood glucose levels and the plasma insulin levels, the exogenous GLP-1(7-36) amide had supposedly disappeared from the body by then. During the test period, no significant difference was observed in the body weight or the food intake among the groups.

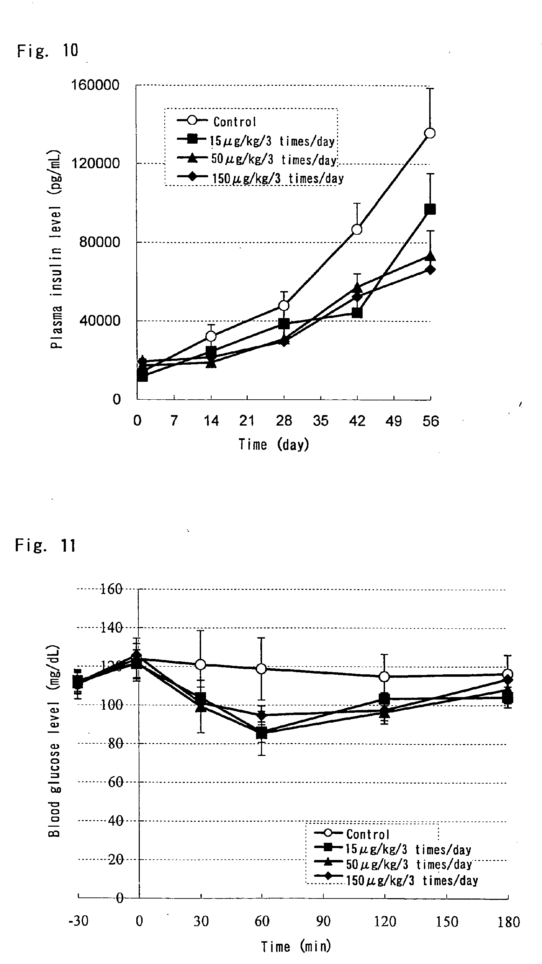

(b) KK-Ay/Ta Jcl mice were subcutaneously administered GLP-1(7-36) amide 3 times daily (at around 9, 13 and 17 o'clock) for about 8 weeks. Following the final administration (at around 17 o'clock), the animals were fasted for more than 16 hours. Subsequently, the mice were intraperitoneally administered 0.75 U/kg of insulin. The blood glucose levels were measured over time. The results are shown in Fig. 11.
The hypoglycemic effect of insulin was enhanced in each of the GLP-1(7-36) amide groups as compared to the control group although the fasting blood glucose levels following the fasting period of more than 6 hours were substantially the same in all groups.

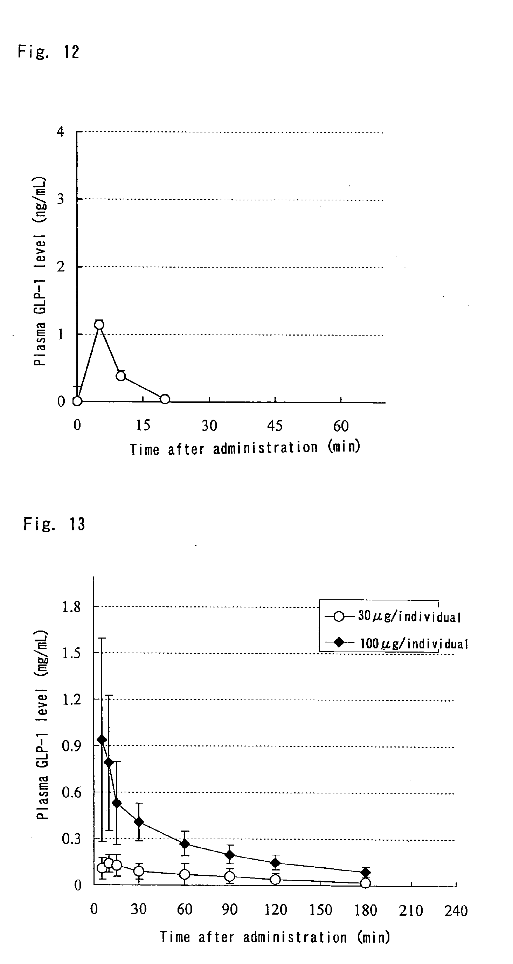

(c) GLP-1(7-36) amide was dissolved in physiological saline and the resulting solution was subcutaneously administered to healthy mice (C57BL mice) at a dose of 50 µg/kg. The changes in the plasma levels of active GLP-1 were monitored. The plasma level of active GLP-1 increased to 1.14 ng/mL 5 minutes after the administration and decreased to an undetectable level (less than 0.1 ng/mL) 30 minutes after the administration (Fig. 12).

### (3) Conclusion

These results indicate that the temporary increase in the plasma levels of active GLP-1 caused by the repeated subcutaneous administration of GLP-1(7-36) amide prevents worsening of insulin resistance and enhances the insulin sensitivity in KK-Ay/Ta Jcl mice. This effect was observed to substantially the same degree in all of the groups administered 15 to 150 µg/kg of GLP-1(7-36) amide 3 times daily, indicating that the effect is saturated at a dose of 15 µg/kg. No significant difference was observed among the groups in the body weight or food intake during the administration period, or in the fasting blood glucose levels after the repeated administration. The administration of GLP-1(7-36) amide caused only minor suppression of the increase in the postprandial blood glucose levels. These observations suggest that GLP-1(7-36) amide enhances the insulin sensitivity by directly improving the insulin resistance of the peripheral tissue: the enhancement is not a secondary effect of hypoglycemic effect or suppression of food intake.

### Example 3: Changes in the plasma levels of active GLP-1 in male cynomolgus monkeys nasally administered a powder nasal preparation of GLP-1(7-36) amide

A single capsule containing a powder nasal preparation of GLP-1(7-36) amide was mounted on a designated device and the drug powder within the capsule was sprayed into the nasal cavity through the nozzle of the device. The changes in the plasma levels of active GLP-1 were monitored.

### (1) Groups

A 30 mg drug powder containing 30 µg or 100 µg GLP-1(7-36) amide was sprayed into the nasal cavity of four cynomolgus monkeys.

### (2) Evaluation and Results

Using a commercially available ELISA kit, the plasma levels of active GLP-1 were measured after the administration of the powder nasal preparation of GLP-1(7-36) amide. As shown, the plasma level of active GLP-1 increased to about 0.15 to about 1.0 ng/mL immediately after administration and decreased to the initial level (before administration) 180 minutes (3 hours) after administration (Fig. 13).

### (3) Conclusion

In this example, the pharmacokinetics of GLP-1(7-36) amide administered in a powder nasal preparation was examined in cynomolgus monkeys. The results suggest that nasally administering the same preparation intermittently or repeatedly for a predetermined period of time can create a circadian variation in the plasma levels of GLP-1(7-36) amide similar to the circadian variation of the endogenous GLP-1(7-36) amide in a healthy human. Accordingly, a method has been established to administer a GLP-1 receptor agonist to human subjects in a non-invasive manner.

The results of the foregoing examples demonstrate that the worsening of insulin resistance in KK-Ay/Ta Jcl mice can be suppressed and hyperinsulinemia and hyperglycemia caused by relative insulin depletion associated with insulin resistance can be improved by intermittently or repeatedly causing a temporary increase in the plasma concentration of a GLP-1 receptor agonist.
The results also demonstrate that nasal administration of a powder nasal preparation of GLP-1(7-36) amide to a cynomolgus monkey can cause a temporary increase in the plasma levels of active GLP-1. This suggests that such a powder nasal preparation of GLP-1(7-36) amide can serve as a non-invasive means to repeatedly administer natural GLP-1(7-36) amide to a human subject upon each meal.

### Example 4: Activity of GLP-1(7-36) amide subcutaneously and repeatedly administered to KK/Ta Jcl mice kept on a high fat diet

GLP-1(7-36) amide was dissolved in physiological saline. The resulting solution was subcutaneously and repeatedly administered to male KK/Ta Jcl mice (Japan Clea) 3 times daily for 15 weeks. The effects on body weight, food intake, baseline blood glucose levels, glycosylated hemoglobin (HbA1c) levels and plasma insulin levels were examined during the administration period. After the 15-week administration period, the animals were switched from the high fat diet to a normal diet, and then back to the high fat diet and the changes in the blood glucose levels were observed. Also, the administration of GLP-1(7-36) amide was discontinued for 2 weeks and the changes in the blood glucose levels were observed. After completion of the administration (22 to 24 weeks after administration was started), livers and hearts were excised. The liver was weighed and observed for the degree of fatty liver. The heart was evaluated for the cardiac function by Langendorff's perfusion technique. The animals were kept under a 12h light/12h dark circadian cycle (light phase: 8 - 20 o'clock) and fed a normal diet (solid diet (CRF-1), Oriental Yeast) or a high fat diet (solid diet (Quick Fat), Japan Clea) during the repeated administration period.

### (1) Groups

The normal diet group and the high fat diet group were each divided into two subgroups: A control group administered the medium (physiological saline) and a test compound group (N = 8-9). The test compound group was subcutaneously and repeatedly administered 150 µg/kg of GLP-1(7-36) amide 3 times daily (at around 9, 13 and 17 o'clock) for 22 to 24 weeks (including the two-week discontinuation period).

### (2) Evaluation and Results

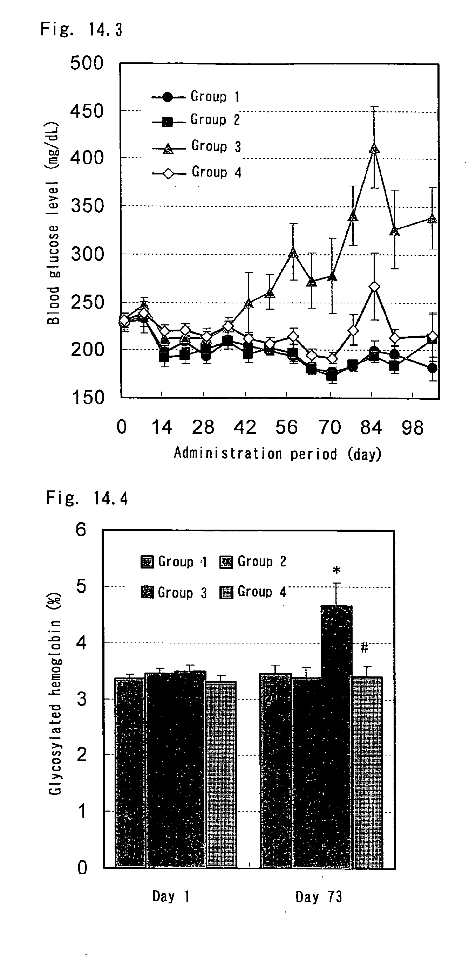

(a) The animals were subcutaneously and repeated administered GLP-1(7-36) amide 3 times daily for 15 weeks and were measured for the body weight, food intake, random blood glucose levels (at 9 and 17 o'clock), glycosylated hemoglobin (HbA1c, at 9 o'clock on Day 1 and Day 73) levels and plasma insulin levels (at 9 o'clock) during the administration period (Fig. 14). In the high fat diet groups, administration of GLP-1(7-36) amide significantly suppressed the increase in the random blood glucose levels (at 9 o'clock) and the plasma insulin levels (at 9 o'clock). For the normal diet groups, no significant difference was observed in the blood glucose levels (at 9 and 17 o'clock) or the HbA1c value between the medium group and the GLP-1(7-36) amide group. For the normal diet groups and high fat diet groups, no significant difference was observed in the food intake between the medium group and the GLP-1(7-36) amide group whereas the increase in the body weight was suppressed in the GLP-1(7-36) amide group.

(b) The animals were subcutaneously and repeatedly administered GLP-1(7-36) amide 3 times daily for 15 weeks and the acetoacetic acid levels were measured (before administration at 9 o'clock on Day 85) (Fig. 15). The acetoacetic acid levels increased significantly in the high fat diet groups as compared to the normal diet groups. The administration of GLP-1(7-36) amide suppressed the increase in the acetoacetic acid levels.

(c) After the 22- to 24-week administration period, the animals were fasted for one night and livers were collected. The liver weight increased significantly in the high fat diet groups as compared to the normal diet groups. Significant fat deposition was observed in the liver of the high fat diet groups. In comparison, the liver weight did not change in the normal diet group administered GLP-1(7-36) amide. The increase in the liver weight was significantly suppressed in the high fat diet group administered GLP-1: little fat deposition was observed in this group (Fig. 16).

(d) After the 22- to 24-week administration period, hearts were collected from the high fat diet groups and evaluated for the cardiac function by Langendorff's perfusion technique. The GLP-1(7-36) amide group showed a faster postischemic recovery of cardiac output than the medium group. Also, the difference between the maximum and minimum perfusion pressure, an index of heart contractility, was larger in this group (Fig. 17).

(e) When the diet of the high fat diet group was switched to a normal diet after the 15-week administration period, the high blood glucose level induced by the high fat diet was immediately decreased. However, the blood glucose level increased to the original level when the diet was switched back to the high fat diet. The blood glucose level of the GLP-1(7-36) amide group did not vary regardless of the type of the diet, nor did it increase rapidly after the 2-week discontinuation period (Fig. 18).

### (3) Conclusion

The results of this example demonstrate that the high fat diet-induced hyperglycemia and hyperinsulinemia in KK/Ta Jcl mice can be improved by subcutaneously and repeatedly administering GLP-1(7-36) amide to the mice to cause intermittent, repeated and temporary increases in the plasma levels of active GLP-1. The generation of acetoacetic acid, a ketone body, and fatty liver are also prevented. The observation that hyperglycemia and hyperinsulinemia were improved after discontinuation of GLP-1(7-36) amide (Fig. 18) suggests that GLP-1 has an ability to balance the glucose/lipid metabolism in liver and to thereby improve insulin resistance and prevent the onset of diabetes. The GLP-1(7-36) amide group showed an improved postischemic cardiac function as compared to the group fed the high fat diet alone. Since the reperfusion was performed more than 12 hours after termination of GLP-1(7-36) amide and the perfusate used did not contain GLP-1, the improved postischemic cardiac function is not due to the traditionally known heart protective function of continuously administered GLP-1. Rather, it is considered that the intermittent, repeated and temporary increases in the plasma levels of active GLP-1 improved insulin resistance of heart and, as a result, the heart muscles acquired resistance to ischemic stress.

### Example 5: Activity of GLP-1(7-36) amide subcutaneously and repeatedly administered to mice kept on high fat diet

Male CRj:CD1(ICR) mice (Charles River Japan) were subcutaneously and repeatedly administered a medium (physiological saline) or 150 µg/kg of GLP-1(7-36) amide twice daily (once daily on holidays) for about 5 weeks. The effects on body weight, food intake, baseline blood glucose levels, and plasma glucose levels and plasma insulin levels after glucose loading were examined during the administration period. The animals were kept under a 12h light/12h dark circadian cycle (light phase: 21 - 9 o'clock) and fed a normal diet (solid diet (CRF-1), Oriental Yeast) or a high fat diet (solid diet (Quick Fat), Japan Clea) during the repeated administration period.

### (1) Groups

Animals were divided into three groups: a control group (N = 6-7) administered the medium and fed the normal diet, another control group (N = 6-7) administered the medium and fed the high fat diet, and a test compound group (N = 6) subcutaneously and repeatedly administered 150 µg/kg of GLP-1(7-36) amide twice daily (at around 9 and 17 o'clock, once daily on holidays) for about 5 weeks.

### (2) Evaluation and Results

The animals were measured for the body weight, food intake and random blood glucose levels (measured before administration at 9 o'clock) during the feeding period. After the final administration, the animals were fasted for one night and were subsequently administered 1.5 g/kg glucose. The plasma glucose levels and plasma insulin levels were measured after 30 minutes.

### (a) Body weight and energy intake

The body weight of the high fat diet group remained greater than that of the normal diet group throughout the feeding period. Administration of GLP-1(7-36) amide suppressed the increase in the body weight (Fig. 19). No significant difference was observed in the weight of food intake (average for each group) among the groups. The food intake for the high fat diet group as measured in energy intake was greater than that for the normal diet group by 10 to 20% (Fig. 20).

### (b) Blood glucose levels

No significant difference was observed in the random blood glucose levels among the groups during the repeated administration period (Fig. 21). The fasting blood glucose levels measured after the one night fasting following the final administration was significantly higher in the high fat diet group than in the normal diet group. Administration of GLP-1(7-36) amide decreased the fasting blood glucose levels (Fig. 21). The plasma glucose level and the plasma insulin level measured 30 minutes after administration of 1.5 g/kg glucose was significantly higher in the high fat diet group than in the normal diet group. Although the increase in the plasma insulin level tends to be suppressed by the administration of GLP-1(7-36) amide, no significant difference was observed between the groups. GLP-1(7-36) amide did not suppress the increase in the plasma glucose levels in the high fat diet group (Fig. 22).

### (3) Conclusion

Repeated administration of GLP-1(7-36) amide to mice kept on a high fat diet suppressed the increase in the body weight resulting from the high fat diet although the food intake did not change. Administration of GLP-1(7-36) amide also suppressed the increase in the fasting blood glucose levels caused by the long-term feeding of high fat diet to substantially the same degree observed in the normal diet group. The high plasma insulin levels caused by high fat diet was also suppressed by GLP-1(7-36) amide. These results are consistent with the results obtained in KK/Ta Jcl mice. This experiment suggests that insulin resistance and obesity can be induced in animals that are not genetically susceptible to diabetes as opposed to KK/Ta Jcl mice and such insulin resistance and obesity can be improved by causing intermittent, repeated and temporary increases in the plasma levels of active GLP-1.

### INDUSTRIAL APPLICABILITY

The present invention provides a novel dosage regimen of GLP-1 receptor agonists that causes little or no side effects or drug interactions and is suitable for improving insulin resistance, as well as an insulin resistance improver for use in the dosage regimen. Specifically, the present invention provides a novel method for improving insulin resistance; a method for preventing or treating hyperglycemia, diabetes, obesity and other disorders that are associated with insulin resistance; and an insulin resistance improver for use in the method containing as an active ingredient a GLP-1 receptor agonist.

## Claims

1. A pharmaceutical composition for improving insulin resistance, or for preventing or treating disorders associated with insulin resistance, the composition containing as an active ingredient a GLP-1 receptor agonist or a pharmaceutically acceptable salt thereof and intended for use on a continuous schedule involving administering the GLP-1 receptor agonist or a pharmaceutically acceptable salt thereof to an individual which exhibits insulin resistance in a single dose of 0.5 to 5000 pg at least once daily before eating for at least 2 weeks.

2. The pharmaceutical composition according to claim 1, wherein the composition is also administered after eating.

3. The pharmaceutical composition according to claim 1 or 2, wherein the GLP-1 receptor agonist is GLP-1(7-36) amide.

4. The pharmaceutical composition according to claims 1 to 3, wherein the composition is administered either subcutaneously, nasally or pulmonarily.

5. The pharmaceutical composition according to claims 1 to 4, wherein the disorder associated with insulin resistance is hyperinsulinemia, diabetes, obesity, hyperlipidemia, arteriosclerosis, hypertension or a cardiovascular disease.

6. The pharmaceutical composition according to claim 7, wherein the disorder associated with insulin resistance is diabetes.

7. The pharmaceutical composition according to claim 1, intended for nasally administering GLP-1(7-36) amide before eating.

8. A method for improving insulin resistance, or for preventing or treating disorders associated with insulin resistance, the method comprising a continuous schedule involving administering a GLP-1 receptor agonist or a pharmaceutically acceptable salt thereof to an individual which exhibit insulin resistance in a single dose of 0.5 to 5000 pg at least once daily before eating for at least 2 weeks.

9. The method according to claim 8, wherein the GLP-1 receptor agonist is also administered after eating.

10. The method according to claim 8 or 9, wherein the GLP-1 receptor agonist is GLP-1(7-36) amide.

11. The method according to any of claims 8 to 10, wherein the GLP-1 receptor agonist is administered either subcutaneously, nasally or pulmonarily.

12. The method according to any of claims 8 to 11, wherein the disorder associated with insulin resistance is hyperinsulinemia, diabetes, obesity, hyperlipidemia, arteriosclerosis, hypertension or a cardiovascular disease.

13. The method according to claim 12, wherein the disorder associated with insulin resistance is diabetes.

14. The method according to claim 8 for nasally administering GLP-1(7-36) amide before eating.

15. A use of GLP-1 receptor agonist in the production of a pharmaceutical composition for improving insulin resistance, or for preventing or treating disorders associated with insulin resistance, the composition containing as an active ingredient a GLP-1 receptor agonist or a pharmaceutically acceptable salt thereof and intended for use on a continuous schedule involving administering the GLP-1 receptor agonist or a pharmaceutically acceptable salt thereof to an individual which exhibit insulin resistance in a single dose of 0.5 to 5000 pg at least once daily before eating for at least 2 weeks.

16. The use according to claim 15, wherein the composition is also administered after eating.

17. The use according to claim 15 or 16, wherein the GLP-1 receptor agonist is GLP-1(7-36) amide.

18. The use according to any of claims 15 to 17, wherein the GLP-1 receptor agonist is administered either subcutaneously, nasally or pulmonarily.

19. The use according to any of claims 15 to 18, wherein the disorder associated with insulin resistance is hyperinsulinemia, diabetes, obesity, hyperlipidemia, arteriosclerosis, hypertension or a cardiovascular disease.

20. The use according to claim 19, wherein the disorder associated with insulin resistance is diabetes.

21. The use according to claim 15 for nasally administering GLP-1(7-36) amide before eating.
